Europäisches Patentamt

European Patent Office

Office européen des brevets

(11) Publication number: **0 267 965**
**A1**

## (12) EUROPEAN PATENT APPLICATION

published in accordance with Art. 158(3) EPC

(21) Application number: **87902742.3**

(22) Date of filing: **21.04.87**

Data of the international application taken as a basis:

(86) International application number: **PCT/JP 87/00253**

(87) International publication number: **WO 87/07263 (03.12.87 87/27)**

(51) Int. Cl.⁴: **C 07 C 57/12,** C 07 C 69/587 // A61K31/20

(30) Priority: **23.05.86 JP 119753/86**

(43) Date of publication of application: **25.05.88 Bulletin 88/21**

(84) Designated Contracting States: **DE FR GB IT NL**

(71) Applicant: **NAKANISHI, Michio, D-502, CI Heights Momoyama-dai, 3-36-8, Higashiizumigaoka, Toyonaka-shi, Osaka 560 (JP)**

(72) Inventor: **NAKANISHI, Michio, D-502, CI Heights Momoyama-dai, 3-36-8, Higashiizumigaoka, Toyonaka-shi, Osaka 560 (JP)**

(74) Representative: **von Kreisler, Alek et al, Patentanwälte Von Kreisler-Selting-Werner Deichmannhaus am Hauptbahnhof, D-5000 Köln 1 (DE)**

(54) **COMPOSITION CONTAINING LINOLENIC ACID COMPOUND.**

(57) A composition containing linolenic acid compound, which further contains at least one phospholipid as a stabilizer for the linolenic acid compound. Linolenic acid compounds have such a poor stability that they will be deteriorated in a short time. In this composition, however, the linolenic acid compounds can stably exist.

EP 0 267 965 A1

00267965

- 1 -

Linolenic Acid Compound-Containing Compositions

Technical Field

This invention relates to stable linolenic acid compound-containing compositions.

Background Art

Linolenic acid is unsaturated fatty acid having 18 carbon atoms and 3 double-bonds.

The effectiveness of $\gamma$-linolenic acid in living organisms has been clarified owing to the recent progress of prostaglandin chemistry. In a normal metabolism route, various species of prostaglandins are formed via $\gamma$-linolenic acid, dihomo-$\gamma$-linolenic acid or arachidonic acid from linolic acid to exhibit their effects. The route of metabolism from linolic acid to $\gamma$-linolenic acid, however, is sometimes hindered. It has been shown that in such a case, it is effective to take $\gamma$-linolenic acid. Thus, linolenic acid, particularly $\gamma$-linolenic acid, has come to draw attention as materials for medicines or health food.

Each species of linolenic acid is an important substance which acts as a precursor for a group of substances such as prostaglandin (PG), thromboxane, and leukotriene. (These have respectively a characteristic physiological activity. For example, PG has a platelet aggregation-inhibiting activity, a vasodilating activity to the arterial walls or the like, and thus an anti-thrombotic action, a preventive action of arteriosclerosis, an anti-cancer action or the like thereof are expected.)  However, since linolenic acid is not produced in human bodies, some human bodies need to be supplied with linolenic acid from outside.

γ-linolenic acid is an intermediate substance produced on the way of conversion of linolic acid to PG.  The conversion of linolic acid to γ-linolenic acid is sometimes disturbed on account of obesity, diabetes, hyperlipoidemia or the like.  Accordingly, it is desirable to take γ-linolenic acid directly.

γ-Linolenic acid had been found so little in nature except one found in mothers' milk.  In the meantime, it was found that γ-linolenic acid is contained in evening primrose seeds and pine tree nuts, and these plants have drawn attention as so-called health food.

However, these linolenic acid and derivatives thereof in the carboxyl group (hereinafter, these are sometimes referred to generically as linolenic acid compounds) are inferior in their stability and have defects of being deteriorated in a short period.

Disclosure of Invention

Thus, the object of this invention is to provide stable compositions of linolenic acid compounds.

The present inventors had conducted extensive studies, and they found that phospholipids have linolenic acid compounds-stabilizing  actions.  It was also found by the present inventors that phospholipids exhibit such stabilizing effects all the better in the presence of a substance which dissolves both linolenic acid compounds and phospholipids.

This invention relates to linolenic acid compound-containing compositions which are characterized in that at least one species selected from among phospholipids is incorporated therein and also relates to the compositions in which a substance that can dissolve both linolenic acid compound and phospholipids is incorporated in said linolenic acid compound-containing compositions.

In the present invention, "linolenic acid compounds" means linolenic acids having 18 carbon atoms and 3 double-bonds (Particularly γ-linolenic acid) and their derivatives in the carboxylic group such as their esters, their acid amides and so on. The esters are exemplified by triglycerides, alkylesters (e.g. straight or branched lower alkylesters having 1 to 4 carbon atoms and so on). As the acid amides, there can be mentioned, for example, dialkyl-amides such as dimethylamides and diethylamides of the linolenic acids, linolenic acid amides, and amides of linolenic acids and amino acids (e.g. linolenyl glycine, linolenyl aniline).

The compositions preferably contain linolenic acid compounds in an amount of not less than 0.1 weight %.

Phospholipids are incorporated in the above-mentioned linolenic acid compounds per se and the product containing the same which are exemplified by evening primrose seeds oil, pine tree nuts and compositions containing as the main ingredient linolenic acid compounds and other ingredients such as diluents which are conventionally used in medicaments and subsidiary health food.

The phospholipids, which are used as a stabilizing agent in the present invention, is a generic name of the lipids containing phosphorus. Some of them have a glyceride as the base skeleton and others do not. They have 1 to 2 fatty acid residues. They are exemplified by phosphatidyl choline, phosphatidyl ethanol amine, sphingomyelin and others. Not only the above-mentioned phospholipids alone but also such modes as their mixture and the phospholipids-containing products are used in the present invention. Particularly, the form of lecitin (e.g. soy bean lecitin, yolk lecitin), hydrogenated lecitin and so on are preferred.

In the compositions of the present invention, phospholipids (e.g. lecitin) is preferably added in an amount of not less than about 0.01 weight parts per 1 weight part of the linolenic acid compounds.

It is preferable that in the compositions of the present invention a substance which dissolves linolenic acid compounds and phospholipids is incorporated in addition to phospholipids. Thus, the linolenic acid compounds are stabilized the better. Said substance is incorporated in such an amount that the linolenic acid compounds and phospholipids can be

- 5 -

solved therein. Said substances are exemplified by vegetable oils [e.g. tocopherol, modified tocopherols (e.g. tocopherol acetate), rice oil, cotton seed oil, corn oil, olive oil, terpenes-containing liquid substances (e.g. extract essence of citrus fruits)], animal oils [e.g. extract from deep-sea sharks (squalene)], organic solvents (e.g. chloroform, dimethylformamide), and protein-disintegrated substances (e.g. collagen).

The phospholipids used in the present invention have an excellent stabilizing action to linolenic acid compounds. The stabilizing action is more remarkably excellent in the presence of a substance which can dissolve the linolenic acid compounds and the phospholipids.

Accordingly, the compositions containing linolenic acid compounds and phospholipids and, in addition thereto, preferably a substance which dissolves linolenic acid compounds and phospholipids, have an effect that linolenic acid compounds can exist in an extremely stable state.

Besides, since γ-linolenic acid and lecitin share physiological activities such as arteriosclerosis-preventing action, anti-thrombotic action, obesity-preventing action and the like, the combined application thereof to the human bodies is particularly favorable in cooperation with the above-mentioned stabilizing action.

Thus, by administering stable compositions comprising linolenic acid compounds of the present invention

- 6 -

to human beings, linolenic acid compounds, particularly linolenic acids, more particularly γ-linolenic acids which cannot be produced naturally in human bodies can be supplied to human bodies from outside in a sufficient amount, and thus a group of substances such as PG, thromboxane and leukotriene, which have characteristic strong physiological activities, can be produced in living organisms.

Examples 1 and 2, Experimental Example 1

In a test tube was put 2 g of extract essence of evening primrose seeds (which contains 7.5 % γ-linolenic acid), and 0.2 g of phosphatidyl choline  or 0.2 g of yolk lecitin was added thereto to give an intimately mixed composition. This mixture was left standing in a thermostatic water bath kept at 70°C without the lid put on. Sampling was conducted periodically and the quantity of the linolenic acid compounds contained therein was estimated in the below-metioned manner. The residual percentage was estimated based on the quantity at 0 hour, which was determined as 100 %. The results are shown in Table 1.

(Quantitative Estimation Method)

To 100 mg of each composition, which was exactly sampled, was added a methanol solution containing 1 % sodium hydroxide to give a 1 ml solution. This solution was vigorously shaken under warming to give a complete solution, the quantity of the linolenic acid compounds of which was estimated as their methyl esters by means of gas chromato-

- 7 -

graphy.

Quantitative Estimation Conditions

: 5% FFAP on Uniport B/2m

Glass column

Temperature of column  205°C

#### Table 1

Residual Percentage of $\gamma$-Linolenic Acid (%)

|  | O hour | 2 days after | 10days after | 30 days after |
|---|---|---|---|---|
| Example 1 (phosphatidyl choline) | 100 | 103 | 102 | 98 |
| Example 2 (yolk lecitin) | 100 | 98 | 102 | 97 |
| Contrast 1 (No phospholipid added) | 100 | 86 | 63 | 35 |

Example 2

Except for using 0.2 g of phosphatidyl choline or 0.2 g of yolk lecitin solved in 0.2 g of vitamine E instead of using 0.2 g of phosphatidyl choline or 0.2 g of yolk lecitin (as a 50 % chloroform solution) in Example 1, the treatment and the test were conducted in accordance with Example 1. The results are shown in Table 2.

#### Table 2

Residual Percentage of $\gamma$-Linolenic Acid (%)

|  | 0 hour | 2 days after | 6 days after | 10 days after | 12 days after | 30 days after |
|---|---|---|---|---|---|---|
| Example 1 (phosphatidyl choline) | 100 | 103 |  | 102 |  | 98 |
| Example 2 (yolk lecitin) | 100 | 98 | 100.5 | 102 | 101.4 | 97 |
| Contrast 1 (No phospholipid added) | 100 | 63 | 51.8 | 43.0 | 36.2 | 24 |

- 8 -

Examples 3 and 4, Experimental Example 2

In accordance with the composition ratio shown in Table
3 (wherein the numerals are shown by weight parts, as is
applied hereinafter), yolk lecitin was dissolved in vitamine
E, and the solution was added to extract essence of evening
primrose seeds (which contains 7.5 % $\gamma$-linolenic acid).  The
mixture was well mixed to obtain a homogeneous composition.
The quantity of linolenic acid compounds was estimated in the
same way as in Experimental Example 1 except that the
temperature of thermostatic water bath was kept at 60°C.  The
results are shown in Table 4.

### Table 3

| | Example 3 | Example 4 | Contrast 3 | Contrast 4 |
|---|---|---|---|---|
| Extract Essence of Evening Primrose Seeds | 100 | 100 | 100 | 100 |
| Vitamin E | 5 | 2 | 5 | – |
| Yolk Lecitin | 5 | 2 | – | – |

### Table 4

Residual Percentage of $\gamma$-Linolenic Acid (%)

| | 0 hour | 6 days after | 12 days after |
|---|---|---|---|
| Example 3 | 100 | 98.0 | 101.5 |
| Example 4 | 100 | 97.7 | 101.2 |
| Contrast 2 | 100 | 75,1 | 66.5 |
| Contrast 3 | 100 | 55.0 | 41.8 |

Examples 5 and 6, Experimental Example 3

In accordance with the composition ratio shown in Table

- 9 -

5, yolk lecitin was dissolved in rice oil, and the solution was added to $\gamma$-linolenic acid. The mixture was well mixed to give a homogeneous composition. The quantity of linolenic acid compounds was estimated in the same manner as in Experimental Example 1 except that the temperature of the thermostatic water bath was kept at 60°C. The results are shown in Table 6.

Table 5

|  | Example 5 | Example 6 | Contrast 4 | Contrast 5 |
|---|---|---|---|---|
| $\gamma$-Linolenic acid | 100 | 100 | 100 | 100 |
| Yolk lecitin | 5 | 2 | - | - |
| Rice oil | 5 | 3 | 5 | - |

Table 6

Residual Percentage of $\gamma$-Linolenic Acid (%)

|  | 0 hour | 6 days after | 12 days after |
|---|---|---|---|
| Example 5 | 100 | 99.6 | 101.5 |
| Example 6 | 100 | 101.3 | 98.6 |
| Contrast 4 | 100 | 78.3 | 57.6 |
| Contrast 5 | 100 | 58.1 | 38.8 |

Examples 7 and 8, Experimental Example 4

In accordance with the composition ratio shown in Table 7, yolk lecitin was dissolved in squalene, and the solution was added to extract essence of evening primrose seeds (which contains about 8 % $\gamma$-linolenic acid). The mixture was intimately mixed to give a homogenous composition. The quantity of linolenic acid compounds was estimated in the same manner as in Experimental Example 1 except that the temperature of the thermostatic water bath was kept at 60°C. The results are shown in Table 8.

Table 7

| Evening primrose | Example 7 | Example 8 | Contrast 6 | Contrast 7 |
|---|---|---|---|---|
| seed extract | 100 | 100 | 100 | 100 |
| Yolk lecitin | 2 | 3 | - | - |
| Squalene | 5 | 10 | 5 | - |

Table 8

Residual Percentage of $\gamma$-Linolenic Acid(%)

| | 0 hour | 6 days after | 12 days after |
|---|---|---|---|
| Example 7 | 100 | 101.8 | 100.9 |
| Example 8 | 100 | 99.2 | 102.1 |
| Contrast 6 | 100 | 72.5 | 38.5 |
| Contrast 7 | 100 | 80.1 | 46.8 |

Experimental Example 5

After the compositions obtained according to Example 3 and Contrast 3 were left standing without the lid being put on in the thermostatic water bath the temperature of which was kept at 60°C, the sampling was conducted periodically. The quantity of the peroxide contained therein was estimated and the peroxide values were determined. The results are shown in Table 9.

Method of the Quantitative Estimation of Peroxide and Calculation of Peroxide Value

In a conical flask with stopper was put exactly 10 g of sample, and 60 ml of solvent (chloroform 2 : glacial acetic acid 3) was added thereto. The mixture was softly shaken to give a transparent solution.

As soon as 1 ml of a saturated potassium iodide solution was added thereto, the stopper was put on. After 1 minute's shaking, 60 ml of water was added and titration was conducted with a 0.01 N sodium thiosulfate solution using a starch solution as an indicator. The vanishing point of coloration by starch is made a terminal point. Simultaneously, a contrast test was conducted. Peroxide value was calculated, based on the following scheme.

Peroxide value (mg equivalent/kg) $= (A - B) \times F \times 10 \div C$

A = the amount of the sodium thiosulfate solution used in the present test (ml)

B = the amount of the sodium thiosulfate solution used in the contrast test (ml)

C = the amount of the sample (g)

F = factor of the sodium thiosulfate solution

Table 9

Residual Percentage of Peroxide (%)

|  | 0 hour | 3 days after | 6 days after |
|---|---|---|---|
| Example 3 | 0.1 | 2.1 | 6.8 |
| Contrast 3 | 1.8 | 7.1 | 11.3 |

Example 9, Experimental Example 6

In accordance with the composition ratio shown in Table 10, yolk lecitin was dissolved in vitamine E, and the solution added to ethylester of $\gamma$-linolenic acid (which contains about 73 % $\gamma$-linolenic acid). The mixture was intimately mixed to give a homogeneous composition. The composition thus obtained was treated in the same conditions as in Experimental Example 5. The quantity of peroxide thereof was estimated and peroxide value (POV) was calculated. The results are shown in Table 11.

Table 10

|  | Example 9 | Contrast 8 |
|---|---|---|
| $\gamma$-Linolenic acid ethylester | 100 | 100 |
| Yolk lecitin | 5 | - |
| Vitamine E | 5 | 5 |

Table 11

Residual Percentage of Peroxide (POV)

|  | 0 hour | 3 days after | 6 days after | 30 days after |
|---|---|---|---|---|
| Example 9 | 0.3 | 1.8 | 3.5 | 5.3 |
| Contrast 8 | 0.5 | 8.3 | 15.2 | 25.2 |

Claims

1. A linolenic acid compound-containing composition characterized in that it comprises at least one species selected from among phospholipids as a linolenic acid compound-stabilizing agent.

2. A linolenic acid compound-containing composition as claimed in Claim 1, wherein the linolenic acid compound is at least one species selected from among γ-linolenic acid and γ-linolenic acid ester.

3. A linolenic acid compound-containing composition as claimed in Clainm 1, wherein the linolenic acid compound is a linolenic acid or in a form wherein it is contained in evening primrose seed oil or pine tree oil.

4. A linolenic acid compound-containing composition as claimed in Claim 1, wherein the phospholipid is in a form of lecitin or hydrogenated lecitin.

5. A linolenic acid compound-containing composition as claimed in Claim 1, wherein the phospholipid is at least one species selected from phosphatidyl choline, phosphatidyl ethanol amine and sphingomyelin.

6. A linolenic acid compound-containing composition as claimed in Claim 1, wherein it comprises a substance which dissolves linolenic acid compounds and phospholipids.

7. A linolenic acid compound-containing composition as claimed in Claim 6, wherein the substance which dissolves linolenic aicd compounds and phospholipids is at least one species selected from vitamine E, squalene and rice oil.

## INTERNATIONAL SEARCH REPORT

00267965

International Application No | PCT/JP87/00253

---

**I. CLASSIFICATION OF SUBJECT MATTER** (if several classification symbols apply, indicate all) [3]

According to International Patent Classification (IPC) or to both National Classification and IPC

Int.Cl[4]  C07C57/12, C07C69/587 //A61K31/20

---

**II. FIELDS SEARCHED**

| Minimum Documentation Searched [4] | |
|---|---|
| Classification System | Classification Symbols |
| IPC | C07C57/12, C07C69/587, A61K31/20 |

| Documentation Searched other than Minimum Documentation to the Extent that such Documents are Included in the Fields Searched [5] |
|---|
| |

---

**III. DOCUMENTS CONSIDERED TO BE RELEVANT [14]**

| Category [*] | Citation of Document, [16] with indication, where appropriate, of the relevant passages [17] | Relevant to Claim No. [18] |
|---|---|---|
| A | JP, A, 61-23699 (Kanegafuchi Chemical Industry Co., Ltd.) 1 February 1986 (01. 02. 86) (Family: none) | 1-7 |

---

* Special categories of cited documents: [16]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

---

**IV. CERTIFICATION**

| Date of the Actual Completion of the International Search [2] | Date of Mailing of this International Search Report [2] |
|---|---|
| July 1, 1987 (01. 07. 87) | July 13, 1987 (13. 07. 87) |
| International Searching Authority [1] | Signature of Authorized Officer [20] |
| Japanese Patent Office | |

Form PCT/ISA/210 (second sheet) (October 1977)